Europäisches Patentamt

(19) European Patent Office

Office européen des brevets

(11) Publication number: **0 456 527 A1**

# (12) EUROPEAN PATENT APPLICATION

(21) Application number: **91400657.2**

(22) Date of filing: **11.03.91**

(51) Int. Cl.⁵: **G01N 33/00, G01N 1/28**

(30) Priority: **06.04.90 US 505407**

(43) Date of publication of application:
**13.11.91 Bulletin 91/46**

(84) Designated Contracting States:
**DE FR GB**

(71) Applicant: **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cédex 07 (FR)**

(72) Inventor: **Brandt, Michael D.**
**4244, North Central Park**
**Chicago, Illinois 60618 (US)**

(74) Representative: **Vesin, Jacques et al**
**L'Air Liquide Service des Relations Industrielles 75, quai d'Orsay**
**F-75321 Paris Cédex 07 (FR)**

(54) **Gas generating apparatus.**

(57)    An apparatus has been devised to continuously generate a constant or other desired dopant level into a gas stream of varying flow rate and composition. A dopant is entrained into a process gas forming a gaseous mixture, the concentration of the dopant in the gaseous mixture is measured, and the flow rate of the dopant is adjusted as a function of the measurement until the desired final dopant concentration is established.

EP 0 456 527 A1

CONTINUOUS MOISTURE GENERATOR

FIG. 3

PROCESS

INLET
50

60

130

55

100
HEATER

32

SATURATOR 70

LEVEL CONTROL

30

PURIFIED WATER
75

SPARGER
80

TEMPERATURE
CONTROLLER

90

MOISTURE
ANALYZER

110

120

MICRO-PROCESSOR

Cross Reference to Related Application

This invention relates to an apparatus which is an improvement of the apparatus disclosed in U.S. application Serial No. 85,888 filed August 14, 1987, now U.S. Patent No. 4,849,174 issued July 18, 1989. The patent is incorporated herein by reference.

Background of the Invention

This invention relates to a gas generating apparatus and more particularly to such an apparatus which continuously generates a constant or other desired moisture level into a gas stream of varying flow rate.

Devices have been developed for emitting a gas, at a more or less constant rate, into a moving fluid medium to produce a known concentration of the gas in the fluid medium. The devices can be used, for example, to facilitate the calibration of certain types of analytical equipment. In these devices, the gas is enclosed in a cylinder under pressure, in equilibrium with its liquid phase or solely in its gaseous phase. The gas permeates through a permeate material to fill an accurately dimensioned passage through one end of the cylinder. In some cases, the cylinder is divided into two chambers, one for holding the gas in liquefied form and the other for holding it in gaseous form. When the cylinder is divided into two chambers, the gas permeates through a membrane between the two chambers and then through another membrane positioned at the exit from the second chamber. Representative devices of this type are disclosed, for example, in U.S. Patents 3,856,204 and 4,399,942.

Heretofore, difficulties have been encountered with the devices of the foregoing type, and these difficulties were of special moment with respect to maintaining the rate of emission of the gas into the fluid medium. As described in the foregoing U.S. Patents, attempts to resolve these difficulties were, for the most part, directed toward maintaining a constant temperature around the cylinder. These attempts were not entirely satisfactory, however, and the use of such prior devices to calibrate many types of analyzers continued to provide inaccurate and nonreproducible results.

Also, it is, of course, well known that there is a need in the semiconductor industry, for example, to accurately control the concentration of water vapor in the gases used during the manufacture of integrated circuits.

Summary

One general object of this invention, therefore, is to provide a new and improved gas generating apparatus which is particularly well suited for accurately providing liquid dopants into process gas in the 0.05 to 100 ppm range.

More specifically, it is an object of the invention to provide a desired low level of moisture in a process gas which varies in both concentration and flowrate.

Another object of the invention is to provide a gas generating apparatus which is economical to manufacture and highly reliable in operation.

In an illustrative embodiment of the invention, the apparatus includes a fluid medium in the form of a supply of inlet or process gas. A mass flow controller is utilized to meter a portion of the inlet gas from the supply and on to a saturator containing a liquid dopant. The gas is put through a sparger in the saturator to break up the gas into fine bubbles to insure saturation of the gas and to achieve the temperature of the liquid dopant. Saturated gas passes from the saturator to the process gas stream to provide a gaseous mixture. The dopant is thus entrained into the process gas. A small portion of the gaseous mixture is sampled and the concentration of dopant in the mixture is measured to determine the desired flow rate through the mass flow controller which will establish the desired final concentration in the gaseous mixture.

The present invention, as well as further objects and features thereof, will become more fully apparent from the following detailed description of certain preferred embodiments, when read with reference to the accompanying drawings.

Brief Description of the Drawings

Fig. 1 is a schematic view of a gas generating apparatus recently made available.

Fig. 2 is a schematic view of a gas generating apparatus for continuously generating dopant into a large flow rate gas stream in accordance with one illustrative embodiment of the invention.

Fig. 3 is a schematic view of a gas generating apparatus for continuously generating dopant into a large flow rate gas stream in accordance with another illustrative embodiment of the invention.

## Description of Certain Preferred Embodiments

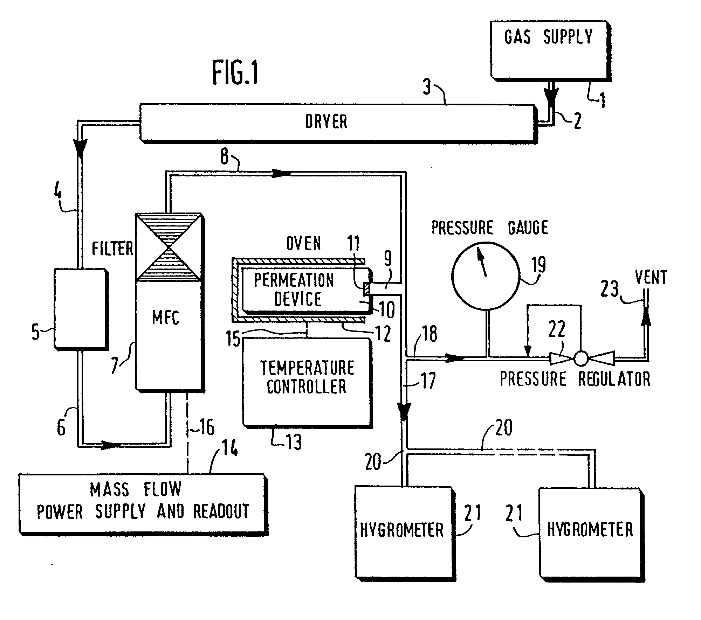

The present invention is an improvement of the apparatus shown in Fig. 1 of the drawings. To appreciate the improvement, Fig. 1 is first described. In Fig. 1, a stream of vector or carrier gas from a gas supply 1 is injected via a duct 2 into a scrubber or dryer 3. The dryer 3 is effective to remove water vapor and other undesired gaseous impurities which may exist in the carrier gas, and it illustratively comprises a 9″ x 1/2″ i.d. cartridge filled with a conventional drying agent. Alternatively, the dryer may be in the form of a 13X or 5A molecular sieve which is activated at an elevated temperature to remove residual moisture.

The carrier gas leaving the dryer 3 is directed through a duct 4 to a filter 5 which serves to remove particulate material that may be contained in the carrier gas stream. In the illustrated embodiment, the filter 5 is a 2μm filter which is provided with a discharge duct 6 for the impurity free carrier gas.

The discharge duct 6 is connected to a mass flow controller indicated schematically at 7. The controller 7 illustratively is of the type available commercially under the trademark ALPHAGAZ-841-09, and it discharges a precise constant flow of gas through a duct 8. The controller 7 is provided with a mass flow power supply and readout device 14 and is connected thereto by a line 16. The device 14 supplies power to the controller 7 and provides an external adjustment to vary the flow rate of the impurity free gas being discharged from the controller.

The duct 8 delivers impurity free gas at a precise massic flow rate from the mass flow controller 7 to a T-shaped mixing junction 9. The central portion of the junction 9 is connected to a permeation device 10 having a membrane 11 for discharging a constant flow of water vapor or other impurity gas into the impurity-free stream of carrier gas as it passes the junction 9. The device 10 illustratively is of the type sold under the commercial reference G-CAL Permeation Device by GC Industries, Inc., and according to the above U.S. Patent 4,399,942, the device includes a dimethyl-polysiloxane membrane.

The permeation device 10 is maintained at a substantially constant temperature by an oven 12 whose temperature is monitored via a line 15 by a temperature controller 13. The controller 13 may be of the type available commercially from OMEGA Engineering, Inc. under the designation OMEGA CN 300 KC. The oven 12 is in the form of an aluminum enclosure having an inner diameter only slightly greater than the external diameter of the device 10 with a heating element such as a belt wrapped around the enclosure and electrically connected through line 15 to the temperature controller 13. The oven maintains the outside temperature of the device within a range of 1°C. of the setpoint temperature to hold the moisture concentration within a few percent of the desired concentration, that is, within the range of 0.01 to 10 ppm. $H_2O$.

For a given membrane 11, at a given temperature, the permeation rate P.R. across the membrane would appear to be a linear function of the pressure P of the carrier gas:

$$P.R. = A (D) + B (D) \times P$$

wherein P is the pressure of the carrier gas sweeping the membrane, and A (D) and B (D) are parameters which are dependent on the flow rate D of the carrier gas past the membrane.

The concentration in the vector gas of impurity gas which permeates through the membrane 11 into the T-junction 9 is given by the following formula:

$$C = \frac{K \times PR}{F}$$

wherein

C = concentration of impurity gas in the carrier gas (in p.p.m. - volume)

K = molar gas constant at 25°C (24.45/molecular weight when F hereunder is in cc/minute at 25°C)

PR = permeation rate of the membrane ($10^{-9}$ g/minute) at a given temperature

F = gas flow rate (cc/minute)

The pressure of the carrier gas stream moving past the membrane 11 is controlled by a pressure regulator 22. The regulator 22 illustratively is of the type available commercially from the Nupro Company under the designation Type R 3A series. The regulator 22 is connected by a duct 18 to the duct 8 on the downstream side of the mixing junction 9 and is provided with a suitable pressure gauge 19. If the pressure of the gas in the duct 8 increases above the predetermined pressure set by the regulator 22, the mixture of carrier gas and impurity gas is vented through a vent 23 until the pressure drops to the predetermined pressure. The pressure of the mixture is thus accurately maintained and closely approximates the predetermined pressure in the vicinity of the membrane 11.

The mixing junction 9 is connected by a duct 17 and duct 20 to one or more hygrometers 21 or other analyzing equipment to be calibrated. The pressure regulator 22 is adjustable to change its pressure threshold and thereby modify the pressure of the mixture in the ducts 8, 17 and 20 and in the junction 9.

The apparatus of Fig. 1 exhibits good results for the calibration of many types of analyzers in the low ppb range.

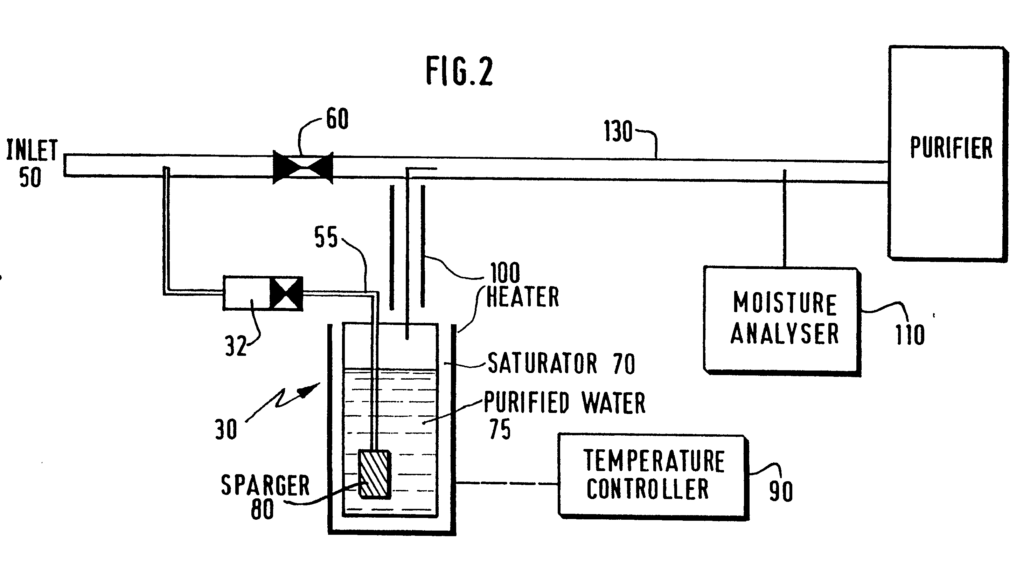

An improvement of the device shown in Fig. 1 is one embodiment of the invention as shown in Fig. 2. This figure represents an embodiment of the invention in which a low part per million level of dopant may be generated continuously into a large flow rate gas even while the gas varies in both composition and flow rate.

Specifically, Fig. 2 is a schematic diagram of a system to generate a desired (e.g., constant) dopant level in a gas of varying flow rate. The process gas enters at the inlet 50 and passes through a restrictor 60 and on to the process. The restrictor creates a pressure drop (proportional to the flow) which provides the differential pressure necessary to drive a moisture level generator 30. The restrictor may be omitted if an independent source of gas, such as a cylinder, is used to drive generator 30. A portion of the inlet gas is then metered off by mass flow control means 32 included in generator 30. The controller 32 is similar to the controller 7 of Fig. 1. The controller 32 discharges the gas through a duct 55 to a saturator 70 (also included in the generator) containing the dopant 75, which may be water or other desired liquid. In the saturator 70, the metered gas passes through a sparger 80 to break up the gas into fine bubbles to ensure saturation of the gas and to maintain a desired temperature of the dopant 75. The dopant level is maintained constant by a dopant level control means (not shown) and the temperature of the dopant is maintained at a preset temperature by temperature control means 90.

Once it is saturated with dopant, the saturated gas passes from the saturator 70 to the process gas stream through a heated transfer line 100, such as a 1/16" tube, to form a gaseous mixture. Transfer line 100 is heated in order to prevent condensation of the dopant and to aid in the mixing of the concentrated saturated gas with the process gas. The dopant is thus entrained into the process gas to form the gaseous mixture which continues down the pipe 130 toward the process. A small portion of the gaseous mixture is sampled by the hygrometer 110 (or other analyzer depending on the nature of the dopant). Then, depending on the concentration of the dopant in the gaseous mixture as measured by the hygrometer, the flow rate through the mass flow controller 32 can be adjusted until the desired final concentration of dopant is established in the gaseous mixture.

This system works well with many gases and takes advantage of a high flow rate to the process and an elevated pressure to produce, for example, a moisture doped gas with a very low level of concentration. The system operates continuously for an indefinite period of time because the dopant level in the saturator is readily maintained constant.

The flow rate through the mass flow controller can be calculated. From this calculation, the flow rate can be initially preset and then adjusted after the concentration of dopant in the gaseous mixture is measured.

The following calculations approximate the set point of the mass flow controller for various gases and flow rates, and using water vapor as the dopant:

For Ar:

Optimum temperature for saturator = 30°C

Concentration parts per million water vapor by volume (ppmv) at 5 kg/cm² and 30°C = 8,240 ppmv

Gas factor for Ar: 1.443

Mass flow controller: 5 sccm ($N_2$)

Flow Range: 8,333 cc/min to 1,667 cc/min

Set point MFC:

For 8,333 cc/min Ar Flow:

$$\text{Set Point} = \frac{2.5 \text{ ppm} \times 8,333 \text{ cc/min} \times 100}{8,240 \text{ ppm} \quad 5 \text{ cc/min} \times 1.443} = 35.0\%$$

For 1,667 cc/min Ar Flow:

$$\text{Set Point} = \frac{2.5 \text{ ppm} \times 1,667 \text{ cc/min} \times 100}{8,240 \text{ ppm} \quad 5 \text{ cc/min} \times 1.443} = 7.0\%$$

For $O_2$:

Otpimum temperature for saturator = 32°C

Concentration parts per million water vapor by volume (ppmv) at 5 Kg/cm² and 32°C = 9,244 ppmv

Gas Factor for $O_2$: .996

Mass flow controller: 5 sccm ($N_2$)

Flow Range: 16,667 cc/min to 3,333 cc/min

For 16,667 cc/min $O_2$ flow:

$$\text{Set point} = \frac{2.5 \text{ ppm} \times 16,667 \text{ cc/min}}{9,244 \text{ ppm}} \times \frac{100}{5 \text{ cc/min} \times .996} = 90.5\%$$

For 3,333 cc/min $O_2$ flow:

$$\text{Set point} = \frac{2.5 \text{ ppm} \times 3,333 \text{ cc/min}}{9,244 \text{ ppm}} \times \frac{100}{5 \text{ cc/min} \times .996} = 13.1\%$$

For $N_2$:

Optimum temperature for saturator = 50°C
Concentration parts per million water vapor by volume (ppmv) at 5 Kg/cm$^2$ and 50°C = 27,833 ppmv
Mass Flow controller:        5 sccm ($N_2$)
Flow range:                 50,000 cc/min to 10,000 cc/min
    For 50,000 cc/min $N_2$ flow:

$$\text{Set point} = \frac{2.5 \text{ ppm} \times 50,000 \text{ cc/min}}{27,833 \text{ ppm}} \times \frac{100}{5 \text{ cc/min}} = 89.3\%$$

For 10,000 cc/min:

$$\text{Set point} = \frac{2.5 \text{ ppm} \times 10,000 \text{ cc/min}}{27,833 \text{ ppm}} \times \frac{100}{5 \text{ cc/min}} = 18.0\%$$

These calculations were made using a single mass flow controller while changing the saturator temperature to accommodate various flow rates. However, changing the flow controller will have the same effect.

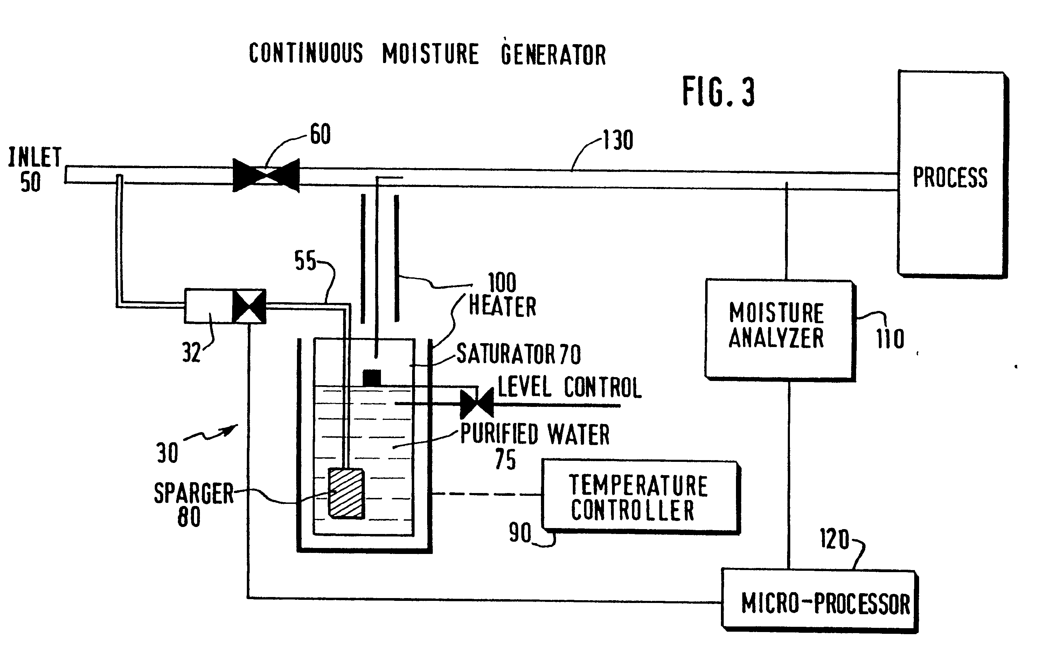

Fig. 3 represents a preferred embodiment of the invention. In a manner similar to that already described with reference to Fig. 2, the dopant is entrained into the process gas forming a gaseous mixture which continues down the pipe 130 toward the process. A small portion of the gaseous mixture is sampled by the analyser 110 and the measured concentration is supplied to a microprocessor 120 (or other electrical controller). The concentration output from the microprocessor 120 is then used as a control signal to control the flow rate through the mass flow controller 32 and thereby establish the desired final concentration of dopant in the gaseous mixture. Further, as the flow rate changes, the final concentration is controlled via the microprocessor to the desired level.

The terms and expressions which have been employed are used as terms of description and not of limitation, and there is no intention in the use of such terms and expressions of excluding equivalents of the features shown and described, or portions thereof, it being recognized that various modifications are possible within the scope of the invention claimed.

## Claims

1. Gas generating apparatus comprising, in combination:

    a supply of carrier gas;

    metering means supplied with carrier gas from said supply for discharging a portion of the carrier gas at a selected flow rate;

    a supply of dopant;

    means for mixing dopant and said carrier gas to provide a doped gas;

    means for adding said doped gas to said carrier gas to form a gaseous mixture;

    means for measuring the concentration of said dopant in said gaseous mixture; and

    means for adjusting said selected flow rate until a desired concentration of dopant in said gaseous mixture is measured.

2. Gas generating apparatus as defined by claim 1 wherein the means for mixing dopant and said carrier gas comprises in combination a saturator and a sparger.

3. Gas generating apparatus as defined by claim 1 wherein the means for adjusting said selected flow rate is an electrical means.

4. Gas generating apparatus as defined by claim 1 wherein said electrical means comprises a microprocessor.

5. Gas generating apparatus as defined by claim 1 wherein said metering means comprises a controllable mass flow controller.

6. Gas generating apparatus as defined by claim 1 wherein said metering means comprises a controllable source of pressurized gas coupled to said means for mixing.

7. A method for continuously generating a constant concentration of dopant into a gas stream of varying composition and flow rate comprising:
   supplying a carrier gas;
   metering a portion of said carrier gas at a selected flow rate;
   supplying a dopant;
   mixing said dopant and said carrier gas to provide a doped gas;
   adding said doped gas to said carrier gas to form a gaseous mixture;
   measuring the concentration of said dopant in said gaseous mixture; and
   adjusting said selected flow rate until a desired concentration of dopant in said gaseous mixture is measured.

8. The method as defined by claim 7 wherein said dopant is water.

FIG.1

EP 0 456 527 A1

FIG.2

EP 0 456 527 A1

CONTINUOUS MOISTURE GENERATOR

FIG. 3

INLET 50

60

32

55

30

SPARGER 80

90

TEMPERATURE CONTROLLER

130

100 HEATER

SATURATOR 70

LEVEL CONTROL

PURIFIED WATER 75

PROCESS

110

MOISTURE ANALYZER

120

MICRO-PROCESSOR

| | European Patent Office | EUROPEAN SEARCH REPORT | Application Number |
|---|---|---|---|

EP    91 40 0657

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | SENSOR AND ACTUATORS. vol. 11, no. 2, March 1987, LAUSANNE CH pages 173 - 186; J.W. GRATE ET AL.: "An automated vapor-generation and data collection instrument for the evaluation of chemical microsensors" * page 174, line 30 - page 179, line 31; figures 1, 2 * * page 183, lines 10 - 38 * --- | 1-8 | G01N33/00 G01N1/28 |
| X | SOLID STATE TECHNOLOGY. vol. 26, no. 6, June 1983, WASHINGTON US pages 45 - 46; PRECISION FLOW DEVICES: "Composition controller." * the whole document * --- | 1-8 | |
| Y | EP-A-0100281 (ARMINES) * page 5, line 16 - page 9, line 13; figures 1, 2 * --- | 1, 3-8 | |
| Y | TECHNISCHES MESSEN. vol. 54, no. 5, 1987, MUENCHEN DE pages 185 - 194; K. MATT ET AL.: "Untersuchung von Gassensoren auf Halbleiterbasis mit einem teilautomatisierten Messplatz" * pages 185-190 :    2 (Messplatz) * --- | 1, 3-8 | TECHNICAL FIELDS SEARCHED (Int. Cl.5 )  G01N |
| A | EP-A-0307265 (L'AIR LIQUIDE) * page 3, line 11 - page 4, line 18; figures 1-4 * | 1, 5-8 | |
| D | & US-A-4849174 ----- | | |

The present search report has been drawn up for all claims

| Place of search | Date of completion of the search | Examiner |
|---|---|---|
| BERLIN | 25 JUNE 1991 | MOUTARD P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)